# EUROPÄISCHE PATENTSCHRIFT

(11) **EP 2 604 292 B1**
(45) Veröffentlichungstag und Bekanntmachung des Hinweises auf die Patenterteilung: **03.03.2021**
(21) Anmeldenummer: 12007795.3
(22) Anmeldetag: 19.11.2012
(51) Int. Cl.: C12M 1/00, A61L 2/06, A61L 2/07, B08B 3/00, B01L 7/00, B01L 3/02, B08B 9/08, B01L 1/02

(54) **Reinigungsverfahren für den Nutzraum eines Brutschranks**
Cleaning method for the useful space of an incubator
Procédé de nettoyage pour l'espace utile d'une étuve

(30) Priorität: 13.12.2011 DE 102011121019
(43) Veröffentlichungstag der Anmeldung: 19.06.2013
(73) Patentinhaber: Thermo Electron LED GmbH, 63505 Langenselbold (DE)
(72) Erfinder: Stahl, Hermann, 61130 Nidderau-Ostheim (DE)
(74) Vertreter: Tomerius, Isabel

(56) Entgegenhaltungen:
- EP-A1- 1 650 291
- EP-A1- 2 359 870
- EP-A1- 2 562 240
- EP-A2- 0 447 256
- EP-A2- 0 923 946
- WO-A1-2005/093038
- US-B1- 6 299 837
- Anonymous: "Backofen reinigen ohne Kraftaufwand | Frag Mutti", , 7 November 2010 (2010-11-07), XP055670508, Retrieved from the Internet: URL:https://www.frag-mutti.de/backofen-rei nigen-ohne-kraftaufwand-a22871/ [retrieved on 2020-02-20]

## Beschreibung

Die Erfindung betrifft ein Verfahren zur Reinigung eines Nutzraums eines Brutschrankes. Brutschränke dienen in Laboratorien üblicherweise dazu, in ihrem Innenraum - nachfolgend als Nutzraum bezeichnet - (mikro)biologische Proben unter vorgegebenen Bedingungen wie einer bestimmten Temperatur und Luftfeuchtigkeit und - im Falle von Begasungsbrutschränken(Inkubatoren) - einer definierten Gasatmosphäre zu lagern. Im Laufe des Betriebs kommt es dabei zu einer Verkeimung des Nutzraumes, die in bestimmten Zeitabständen eine Desinfektion erforderlich macht. Ziel dieser Desinfektion ist es, im Nutzraum befindliche Keime wie beispielsweise Bakterien oder Sporen abzutöten.

Im Stand der Technik ist hierfür ein Desinfektionsverfahren bekannt, bei dem trockene und heiße Luft, beispielsweise mit einer Temperatur von mindestens 140 °C, in den Nutzraum eingeleitet wird und dort die Keime abtötet. Ein anderes Desinfektionsverfahren ist in der EP 0 923 946 B1 beziehungsweise der US 6,299,837 B1 beschrieben. In dem mehrstufigen Verfahren wird anstelle der trockenen heißen Luft zum Abtöten der Keime 90 °C heißer Dampf verwendet. Zur Erzeugung des Dampfes wird ein am Boden des Nutzraums befindliches Wasserreservoir mit unterhalb des Nutzraumbodens angeordneten Heizelementen erhitzt. Gleichzeitig werden sämtliche Heizelemente, die die Wände des Nutzraums erwärmen, betrieben, um eine möglichst hohe Luftfeuchtigkeit von mehr als 80 % im Nutzraum zu erreichen. Diese hochfeuchte Innenraumatmosphäre wird etwa 9 Stunden lang aufrechterhalten. Anschließend werden sämtliche Heizelemente abgeschaltet, um den Dampf aus dem Nutzraum auszukondensieren. In der Abkühlphase soll sich das Kondensat im Bodenbereich, also im Bereich des Wasserreservoirs, wieder ansammeln, während Kondensation an Wänden und Innentür des Brutschranks verhindert werden soll. Anschließend wird der normale Betrieb wieder fortgesetzt. Weitere Dampfdesinfektionsverfahren sind beispielsweise der EP 1 650 291 A1, der EP 0 447 256 A2 und der WO 2005/093038 A1 zu entnehmen. Bei diesen Verfahren wird mit überhitztem Wasserdampf und unter Bedingungen gearbeitet, die einer Kondensation von Wasser aus dem Wasserdampf entgegenwirken.

Nachteil der vorstehend beschriebenen Desinfektionsverfahren ist, dass die abgetöteten Keime im Innenraum des Brutschrankes verbleiben. Diese Verunreinigungen haften beispielsweise an den Wänden und Einbauten des Nutzraumes an. Sollen diese Verunreinigungen entfernt werden, ist ein mühsames Reinigen der genannten Bereiche von Hand notwendig.

Aufgabe der Erfindung ist es entsprechend, ein Verfahren zur Reinigung eines Nutzraumes eines Laboratoriums-Brutschrankes anzugeben, bei welchem eine Entfernung von anhaftenden Verunreinigungen von Hand nicht erforderlich ist.

Die Lösung dieser Aufgabe gelingt mit dem Verfahren nach Anspruch 1. Bevorzugte Verfahrensvarianten sind in den Unteransprüchen beschrieben.

Bei dem erfindungsgemäßen Reinigungsverfahren wird durch Erhitzen eines Wasserreservoirs Wasserdampf erzeugt und gleichzeitig Wasserdampf im Nutzraum auskondensiert. Ziel des erfindungsgemäßen Verfahrens ist es, in großem Maß Wasserdampf zu erzeugen und in großer Menge im Nutzraum, konkret an den Wänden und gegebenenfalls vorhandenen Einbauten des Nutzraumes des Laboratoriums-Brutschranks, auszukondensieren, damit der heiße Wasserdampf die an den Wänden des Nutzraumes und den Einbauten wie Probenträgern, Messvorrichtungen etc. anhaftenden Verunreinigungen löst, abschwemmt und dem Wasserreservoir zuführt, von wo sie aus dem Brutschrank entfernt werden können. Die Erfindung nutzt also den heißen Wasserdampf zum Ablösen der Verunreinigungen, die im Nutzraum anhaften, und vermeidet so ein Entfernen von Hand. Das Ablösen mit heißem Wasserdampf hat zudem den Vorteil, dass der Wasserdampf auch in nur schwer zugängliche Bereiche gelangt, die von Hand praktisch nicht gereinigt werden können. Die Erzeugung von Wasserdampf im Nutzraum erfolgt mit einem Überdruck von höchstens 0,5 bar und kann dabei durch wiederholtes oder kontinuierliches Erhitzen des Wassers im Wasserreservoir erfolgen. Bevorzugt wird kontinuierlich Wasserdampf erzeugt und auskondensiert, und zwar zweckmäßig solange, bis alle Verunreinigungen möglichst vollständig von Wänden und Einbauten des Nutzraums entfernt sind. Der hierfür notwendige Zeitraum hängt von der Art des Brutschranks und dem Grad der Verunreinigungen ab, lässt sich aber durch Routineanalysen leicht ermitteln.

Der Unterschied des erfindungsgemäßen Reinigungsverfahrens zu dem Desinfektionsverfahren, das in der EP 0 923 946 B1 beschrieben ist, besteht vor allem darin, dass gezielt und in großem Umfang Wasserdampf an den Wänden und Einbauten des Nutzraumes auskondensiert wird. Zu diesem Zweck wird während des Kondensationsvorgangs zweckmäßig ständig Wasserdampf nachgebildet, beispielsweise indem das Wasserreservoir weiter erhitzt wird. Im Unterschied dazu wird bei dem Verfahren der EP '946 in der Desinfektionsphase zwar Wasserdampf erzeugt, jedoch dient dieser Schritt nicht der Reinigung des Nutzraumes, sondern der Desinfektion und dem Abtöten von Keimen. Zu diesem Zweck wird Wasserdampf in möglichst hoher Konzentration bei 90 °C im Nutzraum gehalten. Hierzu werden sämtliche Heizelemente, nicht nur im Bodenbereich, sondern auch an den Wänden, betrieben. Eine Kondensation des Wasserdampfes findet in dieser Desinfektionsphase daher nicht statt. Während der anschließenden Kondensationsphase, die der Reduzierung der Wasserdampfkonzentration im Nutzraum dient, soll eine Kondensation von Wasser an den Wänden und der Innentür, die den Nutzraum abschließt, ebenfalls möglichst vermieden werden. Die Kondensation soll vielmehr nur im Bodenbereich des Nutzraumes stattfinden. Während der Kondensationsphase unterbleibt daher im Unterschied zur Erfindung eine Aufheizung des Wasserreservoirs und eine Erzeugung neuen Wasserdampfes.

Auch im Falle des erfindungsgemäßen Verfahrens kann der zu reinigende Nutzraum von Wänden umgeben sein, die mit wenigstens einer Heizvorrichtung beheizbar sind. Diese Heizvorrichtung ist dann jedoch nur für den regulären Betrieb vorgesehen, wird aber während des Reinigungsbetriebes nicht betrieben, damit an den Wänden eine ausreichende Kondensation von Wasser stattfinden kann. Um die Kondensation von Wasser an den Wänden zu fördern, kann es erfindungsgemäß sogar vorgesehen sein, die Wände, die den Nutzraum umgeben, während des Reinigungsverfahrens zu kühlen. Handelt es sich bei dem Brutschrank beispielsweise um einen Brutschrank mit Doppelwandungen, kann in einer einfachen Variante Kaltluft in den Spalt zwischen den Wandungen eingeleitet werden und so die den Nutzraum umgebenden Wände abkühlen. Alternativ kann wenigstens eine Kühlvorrichtung vorgesehen sein, die entlang der Außenseite der Nutzraumwände verläuft. Beispielsweise können Peltierelemente oder Kühlrohre zu diesem Zweck vorgesehen sein. Es ist auch möglich, eine für den normalen Betrieb vorgesehene Heizvorrichtung während des Reingungsvorgangs als Kühlvorrichtung zu verwenden, beispielsweise indem anstelle von Heizfluid ein Kühlfluid in die Rohre eingeleitet wird.

Das Wasserreservoir, das zum Erzeugen von Wasserdampf während des Reinigungsverfahrens verwendet wird, kann in einer Möglichkeit im Inneren des Nutzraumes befindlich sein. Beispielsweise wird in an sich bekannter Weise eine Bodenwanne in den Nutzraum eingestellt oder, wie in der EP '946 beschrieben, dient die den Nutzraum umgebende Ummantelung unmittelbar zur Aufnahme des Wasserbades. In einer anderen und derzeit bevorzugten Variante ist das Wasserreservoir außerhalb des Brutschrankes angeordnet, und Wasserdampf wird von außen in den Nutzraum eingeleitet. Diese Möglichkeit hat den Vorteil, dass der Eintrag von Wasserdampf in höherer Konzentration erfolgen kann als im Falle der Beheizung eines Wasserbades im Innenraum des Nutzraumes. Außerdem werden die Wände in geringerem Maße aufgeheizt und die Kondensation in diesen Bereichen so gefördert. Zur Erzeugung des Wasserdampfes kann beispielsweise in an sich bekannter Weise ein Autoklav oder Heißdampferzeuger eingesetzt werden. Bevorzugt erfolgt der Eintrag von Wasserdampf aber, wie dies von der Anmelderin bereits in den früheren Anmeldungen DE 10 2011 111 754.0 und DE 10 2011 114 900.0, allerdings zu einem anderen Zweck, beschrieben wurde. Insgesamt hat das erfindungsgemäße Reinigungsverfahren den Vorteil, dass es mit Brutschränken des Standes der Technik ohne Weiteres und ohne größere Umbaumaßnahmen durchgeführt werden kann. Dies erleichtert die Anwendung des erfindungsgemäßen Verfahrens erheblich. Erfindungsgemäß erfolgt die Bereitstellung von Wasserdampf im Nutzraum in allen beschriebenen Varianten bei Normaldruck oder einem sehr geringen Überdruck von höchstens 0,5 bar, bevorzugt weniger als 0,2 bar. Um den Aufbau eines höheren Drucks im Nutzraum zu verhindern, kann beispielsweise in diesem ein Druckausgleichsventil oder ein vergleichbares Druckausgleichsmittel vorhanden sein.

Ein weiterer großer Vorteil der Erfindung besteht darin, dass die mit dem erfindungsgemäßen Reinigungsverfahren von den Wänden und Einbauten des Nutzraums abgelösten Verunreinigungen sehr einfach aus dem Nutzraum entfernt werden können. Nach Durchführung des Reinigungsverfahrens befinden sich die Verunreinigungen im auskondensierten Wasserdampf gelöst oder mit diesem ausgeschwemmt am Boden des Nutzraumes. Hier können sie entweder mit dem auskondensierten Wasser von Hand entfernt werden, oder - bevorzugt - werden sie aus einem Abfluss aus dem Inneren des Nutzraumes abgelassen, der im Bereich des Bodens des Nutzraumes, dort wo sich das verunreinigte Wasser ansammelt, angeordnet ist. Besonders bevorzugt ist es dabei, das verunreinigte Wasser in einem Behälter aufzufangen. Bevorzugt wird hierfür ein Wegwerfbehälter verwendet, so dass der Benutzer des Brutschrankes mit dem verunreinigten Wasser möglichst gar nicht mehr in Berührung kommt.

Auch wenn während des erfindungsgemäßen Reinigungsverfahrens eine gewisse Desinfektionswirkung aufgrund des erzeugten heißen Dampfes auftreten kann, ist das Ziel des Verfahrens in erster Linie das Ablösen von Verunreinigungen von Wänden und Einbauten des Nutzraumes. Bevorzugt ist es daher, das erfindungsgemäße Verfahren in Kombination mit einem Sterilisationsschritt durchzuführen. Dieser Sterilisationsschritt dient der gezielten und möglichst umfassenden Abtötung von Keimen im Inneren des Nutzraumes. Hierfür kann jedes bereits aus dem Stand der Technik bekannte Sterilisationsverfahren eingesetzt werden. Beispielsweise kann ein feuchter 90 °C-Desinfektionsschritt verwendet werden, wie er in der EP '946 beschrieben ist. Im Anschluss an diesen Desinfektionsschritt schließen sich dann aber nicht die in der EP '946 beschriebenen Kondensations- und Abkühlschritte an, sondern das Reinigungsverfahren, wie es vorstehend beschrieben wurde. Bevorzugt wird jedoch eine Sterilisation mit trockener Heißluft, zweckmäßig mit einer Temperatur von mindestens 140 °C und vorzugsweise von ca. 180 °C, in Kombination mit dem erfindungsgemäßen Reinigungsverfahren angewendet. Die trockene Sterilisation hat den Vorteil, dass mit ihr auch Sporen zuverlässig abgetötet werden.

Bei der Reihenfolge der Verfahrensschritte kann zunächst ein Sterilisationsschritt durchgeführt werden und im Anschluss daran das erfindungsgemäße Reinigungsverfahren. Mit diesem werden dann die während der Sterilisation abgetöteten Keime aus dem Nutzraum entfernt. Falls gewünscht, kann im Anschluss an das erfindungsgemäße Reinigungsverfahren nochmals ein Sterilisationsschritt durchgeführt werden, um die Sterilität des Nutzraumes für die anschließende normale Weiterbenutzung noch weiter zu verbessern. Alternativ ist es möglich, erst das erfindungsgemäße Reinigungsverfahren durchzuführen und im Anschluss daran einen Sterilisationsschritt, insbesondere eine trockene Sterilisation. Da die Reinigung üblicherweise einige Stunden in Anspruch nimmt, bleibt es, um den normalen Betrieb nicht zu sehr zu beeinträchtigen, bevorzugt dem Benutzer überlassen, wann er dieses Verfahren starten möchte. Es ist jedoch möglich, in bestimmten Zeitabschnitten einen Hinweis vorzusehen, dass eine Reinigung erfolgen sollte. Grundsätzlich ist es ebenfalls möglich, den Reinigungsvorgang durch die Steuerung des Brutschrankes automatisch auszulösen. Nach Abschluss der Reinigung kann ein - zum Beispiel optischer oder akustischer - Hinweis ausgegeben werden, dass das die Verunreinigungen enthaltende Wasserreservoir aus dem Nutzraum entfernt werden soll.

Die Erfindung wird nachfolgend anhand von Zeichnungen näher erläutert. In den nicht maßstabsgerechten und schematischen Zeichnungen zeigen
- Fig. 1: einen Brutschrank im Querschnitt zur Durchführung des erfindungsgemäßen Verfahrens;
- Fig. 2: einen Brutschrank im Querschnitt mit angeschlossenem externen Dampferzeuger zur Durchführung des erfindungsgemäßen Verfahrens und
- Fig. 3: einen weiteren Brutschrank im Querschnitt mit einem anderen externen Dampferzeuger zur Durchführung des erfindungsgemäßen Verfahrens.

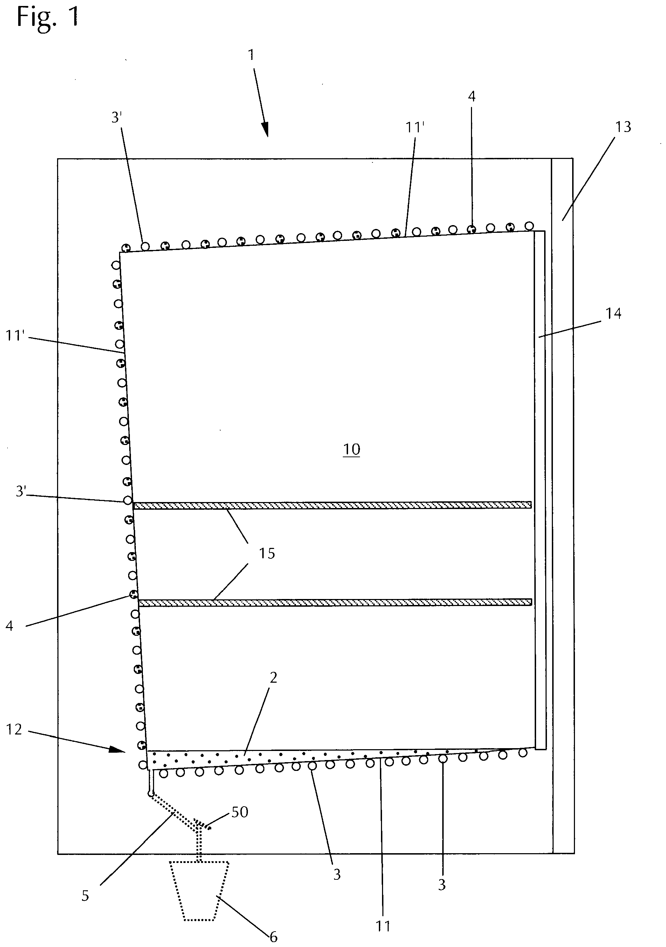

Figur 1 zeigt einen Brutschrank, beispielsweise einen Begasungsbrutschrank oder Inkubator, wie er grundsätzlich bereits aus der EP '946 bekannt ist. Im Inneren des Brutschranks 1, das über eine Tür 13 zugänglich ist, befindet sich ein Nutzraum 10, der von einer Bodenplatte 11 und Wänden 11' begrenzt wird. Der Nutzraum 10 wird zudem von einer Innentür 14 verschlossen, die hinter der Außentür 13 angeordnet ist. Die den Nutzraum 10 umgebenden Wände 11' und die Bodenplatte 11 bilden einen Behälter, der von Innentür 14 weg schräg nach unten geneigt ist. Die Bodenplatte 11 dieses Behälters fällt also von der Innentür 14 schräg nach unten ab. In der auf diese Weise im Bodenbereich 12 gebildeten Senke ist ein Wasserreservoir 2 angeordnet. Das Wasserreservoir 2 kann mithilfe einer Heizvorrichtung 3, hier in Form von Heizrohren, die sich unterhalb der Bodenplatte 11 befinden, beheizt werden, um Wasser aus dem Wasserreservoir zu verdampfen. Heizvorrichtungen 3' sind ebenfalls im Bereich der im Querschnitt nicht erkennbaren Seitenwände sowie von Rück- und Deckenwänden 11' angeordnet. Diese Heizvorrichtungen 3' dient dazu, im normalen Betrieb des Brutschrankes zu verhindern, dass Wasser im Bereich der Wände 11' auskondensiert. Auch die Innentür 14 wird vorzugsweise während des normalen Betriebs des Brutschrankes beheizt, beispielsweise mit einer hier nicht dargstellten Folienheizung. Im Inneren des Nutzraumes 10 sind mehrere Probenträger 15 zur Lagerung von biologischen oder mikrobiologischen Proben oder Ähnlichem vorhanden. Weitere Einbauten im Nutzraum wie beispielsweise Messeinrichtungen usw. sind zur Erhöhung der Übersichtlichkeit nicht gezeigt.

Zur Durchführung des erfindungsgemäßen Reinigungsverfahrens wird der normale Betrieb unterbrochen. Es werden sämtliche Proben aus dem Nutzraum 10 entfernt. Bevorzugt wird zunächst ein Sterilisationsschritt durchgeführt, der beispielsweise im Einleiten von trockener Heißluft bestehen kann. Anschließend wird der Reinigungsbetrieb gestartet. Der gesamte Vorgang wird mittels der Steuerung des Brutschrankes 1 überwacht und gesteuert. Für den Reinigungsbetrieb werden die Heizvorrichtungen 3 im Bereich der Bodenplatte 11 unterhalb des Wasserreservoirs 2 in Betrieb genommen. Dagegen bleiben die Heizvorrichtungen 3' im Bereich der Seitenwände und Deckenwand 11' abgeschaltet. Im Unterschied zum normalen Betrieb sind die Seiten- und Deckenwände 11' also vergleichsweise kalt. Durch Betreiben der Heizung 3 im Bodenbereich 12 wird Wasser aus dem Wasserreservoir 2 verdampft und gelangt als Wasserdampf in den Nutzraum 10. Dieser Wasserdampf kondensiert an den Seitenwänden und der Decke des Nutzraumes 10 aus. Das auskondensierte Wasser fließt an Decke und Seitenwänden 11' ab und zum Bodenbereich 12 zurück und gelangt so wieder in das Wasserreservoir 2. Durch den Heißdampf gelöste Verunreinigungen werden dabei von den Wänden, den Probenträgern 15 und den hier nicht gezeigten weiteren Inneneinbauten des Nutzraumes 10 mitgeschwemmt und reichern sich im Wasserreservoir 2 an. Während des gesamten Reinigungsverfahrens wird die Heizvorrichtung 3 im Bodenbereich 12 des Brutschrankes 1 weiter betrieben, so dass kontinuierlich Wasser aus dem Wasserreservoir 2 verdampft, an den kühlen Seiten- und Deckenwänden auskondensiert und in das Wasserreservoir 2 zurückfließt. Abhängig vom Verunreinigungsgrad des Nutzraumes 10 wird dieser Vorgang solange durchgeführt, bis im Wesentlichen alle abgelagerten Verunreinigungen von Wänden und Einrichtungen des Nutzraumes 10 entfernt sind. Bevorzugt ist es, das Reinigungsverfahren über Nacht laufen zu lassen.

Um die Kondensation an den Seiten- und Deckenwänden 11' weiter zu fördern, ist im gezeigten Fall im Bereich der Seiten- und Deckenwände 11' eine Kühlvorrichtung 4 vorgesehen. Diese Kühlvorrichtung 4 ist hier in Form von Kühlrohren entlang der Außenseiten der den Nutzraum 10 umgebenden Wände 11' ausgebildet. Unterhalb der Bodenplatte 11 dagegen ist keine Kühlvorrichtung 4 vorhanden. Die Kühlung der Seiten- und Deckenwände 11' führt zu einer noch stärkeren Kondensation und zu einer schnelleren Rückführung des verdampften und an den Wänden 11' kondensierten Wassers in das Wasserreservoir 2.

Nach Abschluss der Verdampfungs- und Kondensationsvorgänge und einer Abkühlzeit wird das mit Verunreinigungen angereicherte Wasser aus dem Bodenbereich 12 des Brutschrankes 1 entfernt. In einer einfachen Variante der Erfindung kann dies durch Auswischen des Wasserreservoirs aus dem Nutzraum 10 durch die geöffneten Türen 13 und 14 hindurch geschehen. Bevorzugt ist es jedoch, das Wasser durch einen Auslass 5 aus dem Nutzraum heraus nach außen abzuführen. Im gezeigten Fall befindet sich der Auslass 5 in einem hinteren, von den Türen 13 und 14 abgelegenen und am tiefsten gelegenen Bereich des Bodens des Nutzraumes. Das verunreinigte Wasser wird durch den Abfluss 5 seitlich aus dem Brutschrank 1 heraus nach außen geführt. Die außerhalb der dargestellten Querschnittsebene liegenden Teile des Abflusses sowie des Auffangbehälters 6 sind hier punktiert dargestellt. Aus dem mit einem Hahn 50 verschließbaren Abfluss 5 wird das verunreinigte Wasser direkt in einen Wegwerfbehälter 6 geleitet. Bei diesem Wegwerfbehälter 6 kann es sich beispielsweise um einen mit Watte gefüllten Becher aus Recyclingkunststoff handeln, der im Abfall entsorgt werden kann, ohne dass der Benutzer mit dessen Inhalt in Berührung kommt.

Nach Abschluss des Reinigungsverfahrens kann der normale Betrieb, gegebenenfalls nach Durchführung eines zusätzlichen Sterilisationsschrittes, wieder aufgenommen werden.

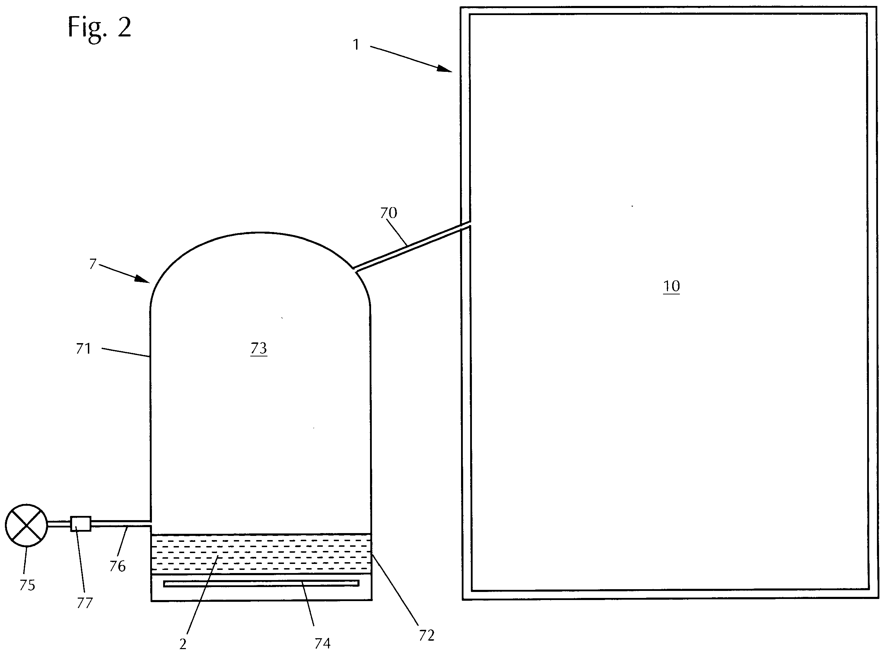

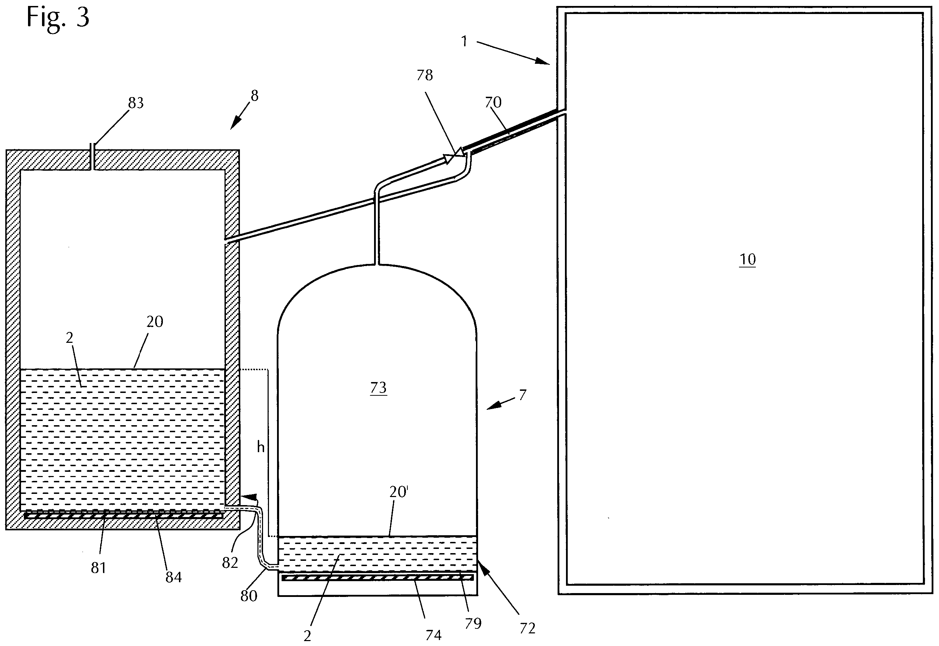

Figuren 2 und 3 beschreiben die Erfindung am Beispiel von Begasungsbrutschränken (Inkubatoren) 1 mit externen Dampferzeugern 7. Der Inkubator 1 kann grundsätzlich wie in Zusammenhang mit Figur 1 beschrieben aufgebaut sein. Die Einzelheiten sind hier der Übersichtlichkeit halber nicht dargestellt. Auch der Verfahrensablauf entspricht grundsätzlich dem vorstehend beschriebenen und unterscheidet sich von diesem lediglich in der Art der Wasserdampferzeugung. Anstelle des Verdampfens von Wasser im Inneren des Nutzraumes 10 wird diesem Wasserdampf von außerhalb zugeführt.

Im Falle von Figur 2 ist ein externer Dampferzeuger 7 über eine Dampfzuleitung 70 mit dem Nutzraum 10 des Inkubators 1 verbunden. Der Dampferzeuger 7 besteht aus einem Behälter 71 mit einem Bodenbereich 72, der ein Wasserreservoir 2 aufnehmen kann, und einem darüber liegenden, hier kuppelförmig ausgebildeten Dampfraum 73. Bei dem Behälter 71 handelt es sich um einen drucklosen Behälter, in dem Wasserdampf, der durch Erhitzen des Wasserreservoirs 2 mittels einer Heizvorrichtung 74 erzeugt wird, im Dampferzeuger 7 nicht komprimiert wird, so dass der Dampfdruck im Wesentlichen dem Umgebungsdruck entspricht und in jedem Fall bei höchstens 0,5 bar, üblicherweise unter 0,2 bar, liegt.

Der im Dampfraum 73 vorhandene Wasserdampf wird dem Inkubator 1 dadurch zugeführt, dass mittels einer Pumpe 75 Luft aus der Umgebung des Dampferzeugers 7 über die Luftzuleitung 76 in das Innere des Dampferzeugers 7 gefördert wird. Im gezeigten Fall mündet die Luftzuleitung 76 oberhalb des Wasserreservoirs 2 in den Dampfraum 73. Es ist alternativ auch möglich, die Luftzuleitung tiefer zu legen, sodass die Umgebungsluft durch das Wasserreservoir 2 hindurch in den Dampfraum 73 geführt wird. Im Dampferzeuger reichert sich die Umgebungsluft mit dem Wasserdampf an und gelangt über die Dampfzuleitung 70 in den Nutzraum 10 des Inkubators. Damit mit der Umgebungsluft keine Verunreinigungen in den Dampferzeuger 7 gefördert werden, ist zwischen Pumpe 75 und Dampferzeuger 7 in der Luftzuleitung 76 ein Sterilfilter 77 angeordnet.

Figur 3 verwendet einen Dampferzeuger 7, der ebenfalls aus einem Behälter besteht, der in einem Bodenbereich 72 ein Wasserreservoir 2 aufnehmen kann. Im Dampferzeuger 7 wird Wasserdampf durch Erhitzen des Wasserreservoirs 2 mittels einer Heizvorrichtung 74 erzeugt. Der Wasserdampf gelangt in den Dampfraum 73 und von dort über eine Dampfzuleitung 70, die den Dampferzeuger am höchsten Punkt des Dampfraumes 73 verlässt, in den Nutzraum 10 des Inkubators 1. Die Dampfzuleitung 70 ist mit einem Ventil 78 verschließbar, das in an sich bekannter Weise geöffnet wird, wenn dem Nutzraum 10 für die Reinigung Wasserdampf zugeführt werden soll.

Der Dampferzeuger 7 ist über eine aus seinem Bodenbereich 72 herausführende Wasserzuleitung 80 mit einem Wasserbehälter 8 verbunden und so angeordnet, dass der das Wasserbad 2 tragenden Boden 79 des Dampferzeugers 7 oberhalb des entsprechenden Bodens 81 des Wasserbehälters 8 liegt. Die Wasserzuleitung 80 führt aus einem Bodenbereich 82 des Wasserbehälters 8 heraus. Das Wasserreservoir 2 verteilt sich so auf den Dampferzeuger 7 und den Wasserbehälter 8. Wird das Wasserreservoir 2 im Dampferzeuger durch Betreiben der Heizvorrichtung 74 auf bevorzugt ca. 100 °C erhitzt, bildet sich Wasserdampf und füllt den Dampfraum 73 an. Dadurch steigt der Druck im Dampferzeuger 7, der Wasserspiegel 20' sinkt und Wasser wird aus dem Dampferzeuger 7 über die Wasserzuleitung 80 in den Wasserbehälter 8 verdrängt. Dort steigt der Wasserspiegel 20 an. Fig. 3 zeigt dies, also einen Zustand während der Erzeugung von Wasserdampf im Dampferzeuger 7. Der Dampfdruck im Dampferzeuger 7 entspricht der Wassersäule, die oberhalb des Wasserspiegels 20' steht, also der Differenz der Wasserspiegel 20 und 20' (Höhe h).

Die Anordnung des Wasserbehälters 8 relativ zum Dampferzeuger 7 ist derart, dass unter normalen Umständen das in der Wasserzuleitung 80 stehende Wasser nicht vollständig aus der Leitung verdrängt wird. Eventuell in die Wasserzuleitung 80 eindringender Dampf kommt mit dem kälteren Wasser in der Leitung 80 in Kontakt, kühlt ab und kondensiert aus, wodurch der Druck absinkt. Zum Ausgleich sehr hohen Überdrucks ist im Wasserbehälter 8 zudem eine Druckausgleichsöffnung 83 vorhanden, durch die eventuell doch in den Wasserbehälter 8 eindringender Dampf entweichen kann. Dampfexplosionen werden auf diese Weise sicher vermieden. Das Wasserreservoir 2 im Wasserbehälter 8 muss prinzipiell gar nicht erwärmt werden. Im gezeigten Fall ist jedoch eine Heizvorrichtung 84 vorhanden, mit der das Wasserreservoir 2 auch im Wasserbehälter 8 erwärmt wird, wenn auch auf eine niedrigere Temperatur als im Dampferzeuger 7. Vorzugsweise wird auf eine Temperatur von mindestens 60 °C, besonders bevorzugt auf 80 bis 90 °C, erhitzt. Je höher die Temperatur des Wasserreservoirs 2, desto geringer die Wahrscheinlichkeit, dass sich Keime im Wasser bilden, und desto geringer die Heizleistung, um Wasser im Dampferzeuger 7 zum Sieden zu bringen. Die Temperatur des Wasserreservoirs 2 im Wasserbehälter 8 sollte jedoch nicht so hoch sein, dass das Wasser dort zu sieden beginnt.

## Patentansprüche

1. Verfahren zur Reinigung eines von Wänden (11') umgebenen Nutzraumes (10) eines Brutschrankes (1), bei dem durch Erhitzen eines Wasserreservoirs (2) Wasserdampf erzeugt und gleichzeitig an den Wänden (11') des Nutzraumes und an gegebenenfalls im Nutzraum (10) vorhandenen Einbauten (15) auskondensiert wird, wobei der Wasserdampf im Nutzraum (10) mit einem Überdruck von höchstens 0,5 bar erzeugt wird, und wobei in einem Maß Wasserdampf erzeugt und im Nutzraum (10) auskondensiert wird, dass der heiße Wasserdampf an den Wänden (11') des Nutzraumes und den Einbauten (15) anhaftende Verunreinigungen löst und abschwemmt.

2. Verfahren nach Anspruch 1, wobei der Nutzraum (10) von Wänden (11') umgeben ist, die mit wenigstens einer Heizvorrichtung (3') beheizbar sind, wobei die Heizvorrichtung (3') während der Reinigung abgeschaltet ist.

3. Verfahren nach Anspruch 1 oder 2, bei dem die Wände (11') während der Reinigung mit wenigstens einer Kühlvorrichtung (4) gekühlt werden.

4. Verfahren nach einem der Ansprüche 1 bis 3, bei dem das Wasserreservoir (2) in einem Bodenbereich (12) innerhalb des Nutzraumes (10) angeordnet ist.

5. Verfahren nach einem der Ansprüche 1 bis 3, bei dem sich das Wasserreservoir (2) außerhalb des Brutschrankes (1) befindet und Wasserdampf in den Nutzraum (10) eingeleitet wird.

6. Verfahren nach einem der Ansprüche 1 bis 5, bei dem der Wasserdampf im Nutzraum (10) mit einem Druck von weniger als 0,2 bar erzeugt wird.

7. Verfahren nach einem der Ansprüche 1 bis 6, bei dem nach Abschluss der Reinigung kondensiertes Wasser im Bodenbereich (12) des Nutzraumes (10) gesammelt und von dort aus dem Nutzraum (10) entfernt wird.

8. Verfahren nach Anspruch 7, bei dem kondensiertes Wasser über einen Abfluss (5) abgelassen und bevorzugt in einem Wegwerfbehälter (6) aufgefangen wird.

9. Verfahren nach einem der Ansprüche 1 bis 8, welches zusätzlich einen Sterilisationsschritt umfasst, bevorzugt einen Sterilisationsschritt, bei dem in den Nutzraum (10) trockene Heißluft eingeblasen wird.

10. Verfahren nach Anspruch 9, bei dem der Sterilisationsschritt nach dem Reinigungsschritt erfolgt oder sowohl vor als auch nach dem Reinigungsschritt ein Sterilisationsschritt ausgeführt wird.

## Claims

1. Method for cleaning a usable space (10) of an incubator (1), which usable space is surrounded by walls (11'), wherein, by heating a water reservoir (2), steam is generated and simultaneously condensed on the walls (11') of the usable space and on fixtures (15) optionally present in the usable space (10), the steam in the usable space (10) being generated at an overpressure of at most 0.5 bar, and steam being generated and condensed in the usable space (10) to the extent that the hot steam dissolves and washes away impurities that adhere to the walls (11') of the usable space and the fixtures (15).

2. Method according to claim 1, the usable space (10) being surrounded by walls (11') which can be heated by at least one heating device (3'), the heating device (3') being switched off during cleaning.

3. Method according to either claim 1 or claim 2, wherein the walls (11') are cooled by at least one cooling device (4) during cleaning.

4. Method according to any of claims 1 to 3, wherein the water reservoir (2) is arranged in a floor region (12) within the usable space (10).

5. Method according to any of claims 1 to 3, wherein the water reservoir (2) is located outside the incubator (1) and steam is introduced into the usable space (10).

6. Method according to any of claims 1 to 5, wherein the steam in the usable space (10) is generated at a pressure of less than 0.2 bar.

7. Method according to any of claims 1 to 6, wherein, after cleaning has been completed, condensed water is collected in the floor region (12) of the usable space (10) and then removed from the usable space (10).

8. Method according to claim 7, wherein condensed water is drained off via a drain (5) and preferably collected in a disposable container (6).

9. Method according to any of claims 1 to 8, which method additionally comprises a sterilization step, preferably a sterilization step in which dry hot air is blown into the usable space (10).

10. Method according to claim 9, wherein the sterilization step occurs after the cleaning step or a sterilization step is performed both before and after the cleaning step.

## Revendications

1. Procédé de nettoyage d'un espace utile (10) d'une étuve (1), lequel espace utile est entouré de parois (11'), de la vapeur d'eau étant générée en chauffant un réservoir d'eau (2) et, simultanément, étant condensée sur les parois (11') de l'espace utile et sur des armatures (15) éventuellement présentes dans l'espace utile (10), la vapeur d'eau dans l'espace utile (10) étant générée à une surpression d'au plus 0,5 bar, et la vapeur d'eau étant générée et étant condensée dans l'espace utile (10) de telle sorte que la vapeur d'eau chaude dissout et élimine les impuretés adhérant aux parois (11') de l'espace utile et aux armatures (15).

2. Procédé selon la revendication 1, dans lequel l'espace utile (10) est entouré de parois (11') qui peuvent être chauffées au moyen d'au moins un dispositif de chauffage (3'), le dispositif de chauffage (3') étant éteint pendant le nettoyage.

3. Procédé selon la revendication 1 ou 2, dans lequel les parois (11') sont refroidies pendant le nettoyage au moyen d'au moins un dispositif de refroidissement (4).

4. Procédé selon l'une des revendications 1 à 3, dans lequel le réservoir d'eau (2) est disposé dans une zone de fond (12) au sein de l'espace utile (10).

5. Procédé selon l'une des revendications 1 à 3, dans lequel le réservoir d'eau (2) est situé à l'extérieur de l'étuve (1) et de la vapeur d'eau est introduite dans l'espace utile (10).

6. Procédé selon l'une des revendications 1 à 5, dans lequel la vapeur d'eau dans l'espace utile (10) est générée à une pression inférieure à 0,2 bar.

7. Procédé selon l'une des revendications 1 à 6, dans lequel, une fois le nettoyage terminé, l'eau condensée est collectée dans la zone de fond (12) de l'espace utile (10) et, à partir de là, est retirée de l'espace utile (10).

8. Procédé selon la revendication 7, dans lequel l'eau condensée est évacuée par l'intermédiaire d'une sortie (5) et de préférence est recueillie dans un récipient jetable (6).

9. Procédé selon l'une des revendications 1 à 8, lequel comprend en outre une étape de stérilisation, de préférence une étape de stérilisation dans laquelle de l'air chaud sec est insufflé dans l'espace utile (10).

10. Procédé selon la revendication 9, dans lequel l'étape de stérilisation est réalisée après l'étape de nettoyage, ou une étape de stérilisation est effectuée aussi bien avant qu'après l'étape de nettoyage.
